# EUROPEAN PATENT APPLICATION

(11) **EP 1 001 618 A1**
(43) Date of publication of application: **17.05.2000**
(21) Application number: 98309219.8
(22) Date of filing: 11.11.1998
(51) Int. Cl.: H04N 5/232, G06F 1/16

(54) **Video camera system**

(71) Applicant: Xybernaut Corporation, Fairfax, Virginia 22033 (US)
(72) Inventor: Newman, Edward G., Fairfax Station, Virginia 22039 (US); Jenkins, Michael D., Burke, Virginia 22015 (US); Schwartz, Steven J., Fairfax, Virginia 22033 (US)
(74) Representative: Goode, Ian Roy

(57) **Abstract**

This invention involves the use of a body worn mobile computer (2) with a video camera (1) to allow image alignment. Quite frequently a video camera user cannot look directly into the camera because of some obstruction. In these cases, in order to be sure the camera is pointed in the right direction and toward the object to be captured by the image, a head mounted display (8) worn by the user and connected (7) to a computer can simplify this process. An example of where this system can be used is when the camera operator is located in the rear of a crowd watching a parade. He or she can hold the camera viewer above the crowd and view (13) the image in their head mounted display thereby moving the camera viewer in the desired direction. The visible image can then be simultaneously displayed (13) to the wearer (or user) and stored or recorded on the wearable computer (2) or even transmitted via the computer peripherals to a remote location.

## Description

This invention relates to a video camera system and, more specifically, to a system for transmitting views from a video camera to a user.

Video capture devices similar to the consumer video cameras have viewfinders that allow a video cameraman to view information that is being captured. This works well in environments that allow the cameraman to hold the video camera close enough to allow the cameraman to look through the viewfinder. However, there are situations that prohibit the cameraman from being able to look through the view finder to confirm that what the camera is recording is what the cameraman is intending on capturing.

There are several situations where a cameraman or woman desires to capture views on a video camera that are not clearly viewable by the user. For example, if a user is located in the rear of a crowd watching a parade and he or she wishes to capture on video elements of the parade, most likely the user will hold the camera above his head in an attempt to have the camera's view avoid the crowd in front of the user. When the user attempts to do this, he has no way of verifying the proper direction to what the camera is actually viewing and his process is hit and miss. The chance of the user in this situation missing what parade view is intended is very likely. In other situations where the user cannot easily observe the view being captured through the viewfinder such as after earthquakes or collapsed buildings, a viewing probe is usually inserted into areas between the rubble. In these cases also it would be very desirable if the user could align what the camera is viewing or verify that the camera is viewing the intended subject matter. Once this is accomplished, the user could alter the angle or point direction of the video camera to ensure that it is capturing the intended image.

At the present time there is no accommodation when the user cannot observe a camera view for the user of a video to view, confirm, align and adjust a view the camera is recording. In U.S. Patent No. 5,305,244 (Newman et al) a hands-free user-supported mobile computer is disclosed. In the Newman et al system, a compact, self-contained portable computing apparatus is provided which is completely supported by a user for hands-free retrieval and display of information for the user. The computing apparatus includes a voice-recognition module, in communication with a processor, for receiving audio commands from the user, for converting the received audio commands into electrical signals, for recognizing the converted electrical signals and for sending the recognized electrical signals to the processor for processing, the voice-recognition module being supported by the user. The computing apparatus further includes a display in communication with the processor for receiving information from the processor and for displaying the received infortnation for the user, the display being supported by the user whereby the user may operate the computing apparatus to display information in a hands-free manner utilizing only audio commands.

It is known in U.S. Patent 5,189,512 to use a helmet integrated display system (HID) to remotely view a video signal where the HID is worn on a cameraman's or camera woman's head. Through a network of mirrors and a double convex lens, the visible image is converted into a virtual image which image is presented to a sighting eye of the camera person. An occlusion device, also attached to the HID system, partially blocks off the view out of a non-sighting eye such that the perceived images from both eyes combine in the cerebral cortex of the camera person's brain into one image. This display system is a simple structure useful only in remotely viewing the video image.

It would be useful if a computer means was included in a system intermediate or in the same system with a HID and a video camera. For example, if a video was being viewed of a site destroyed by fire, earthquake or other destructive force, it could be instantly on the spot compared with any computer generated images before destruction. Another use for this type system would be for motion picture production where focusing or other manipulation of the camera is required where the holder of the camera may not complete these manipulations while his hands are holding the camera. A voice or other hands-free camera manipulation would be extremely desirable. Other reasons for a hands-free computer in this type system are to communicate images and information to other locations and to systems such as Internet etc. Also, if a parade or other activity was being videotaped, computer generated images or sounds could be instantly produced by voice or other hands-free commands on the videotape being formed. In other cases, the processor could record and play back every Nth frame of the image being videotaped to easily align the direction of the camera being used. A user supported or worn mobile hands-free computer would greatly assist the camera person especially when direct viewing through the video camera is not possible. The user supported hands-free computer apparatus described in EP-A-0,767,417 would be invaluable in this type system.

It is therefore an object of this invention to provide a system for a video camera user to view subject matter when he cannot use the camera viewfinder.

Another object of this invention is to provide a system for a video camera user to adjust his camera direction, when the user is not sure what is being seen by the camera.

Yet a further object of this invention is to provide a reliable method for a user to capture and/or transmit live video information from a video camera when the user cannot easily observe the information being captured through the viewfinder.

Still a further object of this invention is to provide a method and system that will improve the accuracy of probes used to locate victims in collapsed buildings and the like.

These and other objects of this invention are accomplished by a system where a mobile computer with a display screen or HMD can be used to view a camera image while not using the camera viewfinder. A preferable mobile computer to be used in the present invention and the activation means is the type disclosed in EP-A-0,767,417 (Mobile Assistant ®), however, any suitable mobile computer with a viewing screen can be used. The Mobile Assistant ® is preferred since it is a hands-free computer system that allows the user to hold a video or other camera in his hands, however, any suitable mobile computer with a viewing screen can be used.

The computer of EP-A-0,767,417 "Mobile Assistant"® or "Mobile Computer" is ideal for use in the present invention because it is hands-free with various activation means allowing the user to hold a video camera while still having all of the advantages provided by a hands-free activated computer. Also, the video camera can be easily connected to the processor of the Mobile Assistant ® so that the camera view is displayed on the HMD (head mounted display) of the Mobile Assistant ®. This method allows the video camera to be moved or manoeuvred into areas that traditionally would be impossible to view while verifying that the intended subject to be videotaped was captured correctly. This is accomplished by having the video capture device attached to the mobile computer and viewing the image being picked up by the video camera. Once the image is viewed as noted, the handheld video camera can be moved until the view desired is displayed on the HMD. The mobile computer then routes the video information to a storage device or to the communications port to transmit the information to an offsite location. While the information is being routed, the computer will grab every Nth frame and display it on the user's computer monitor or head mounted display. Using this method gives the cameraman the ability to observe what is being captured and adjust the position of the camera as necessary.

There are also means to control Nth frames exposure alignment of the subject being captured. By Nth frames is meant any preselected number such as every 5^{th} frame or every 10^{th} frame, etc.

The adaptation of a video camera for use in the present invention is relatively simple. A PCMCIA (PC) card can be used in the processor of the Mobile Assistant ® and this then is connected to the video camera using an RCA jack. The PC card used can be any suitable card such as Card Cam that is available from Quadrant Corporation of Malvern, Pennsylvania.

The present system will be described throughout as using a video camera, however any other type camera such as a still camera can be equally used. Also, any suitable mobile computer other than the Mobile Assistant ® can be used if desired, however the Mobile Assistant ® because of its versatility is highly preferred. The Mobile Assistant ® generally functions as follows.

It uses a compact, self-contained portable computing apparatus which is completely supported by a user for hands-free retrieval and display of information for the user. The computing apparatus includes a housing having securing means for removably securing the housing to a user for support by the user. The housing further includes storage means for storing previously entered information and processor means, communicating with the storage means for receiving, retrieving and processing information and user commands in accordance with a stored program. The computing apparatus also includes audio transducer and converter means in communication with the processor means for receiving audio commands from the user for converting the received audio commands into electrical signals, for recognizing the converted electrical signals and for sending the recognized electrical signals to the processor means, the audio transducer and converter means also being supported by the user. The computing apparatus further includes display means in communication with the processor means for receiving information from the processor means and for displaying the received information for the user, the display means being supported by the user whereby the user may operate the computing apparatus to display information in a hands-free manner utilizing only audio commands.

There are many situations when a person operating a wearable computer will have need for a video camera to focus on an item that is not directly visible to the wearable computer operator. Such an instance would include using the camera with a handheld rod that provides for the camera to be positioned around a visual obstruction such as a device under repair located within an enclosure in a narrow space. The image from the camera would be delivered to the wearable computer where it would be digitized and processed and formatted for distribution within the wearable computer. The visual image can then be simultaneously displayed to the wearable computer operator, stored or recorded on the wearable computer and transmitted to a remote location for analysis.

Such an image/video capture system might consist of a video camera such as the Sony "XC-999" CCD made by Sony Corporation, Tokyo Japan or other cameras that are capable of capturing and/or communicating still images or motion video. The camera could be connected via multi-conductor cable to an image/video capture card such as the "Cardcam Video-in" made by Quadrant International, Malvern, PA or could be communicated via a wireless means to a host computer which contains an image/video capture card. The image/video capture card can be built-in to the host computer, built into the actual camera or it can be inserted as a PCMCIA or "PC Card" into the host computer. In the above-mentioned image/video capture card device, it would be plugged into a PCMCIA compatible slot housed inside a host computer such as the Mobile Assistant ® II made by Xybernaut Corporation, Fairfax VA.

In the above configuration, the image/video capture card would take information from the video camera, convert the analog information to digital and perform one of the following: (1) store the information on the hard drive or other media for later transmitting, (2) directly route all information to a communication means connected to the host computer for transmitting this information to a remote location or (3) both store and communicate the information simultaneously. In any event, the stored program is controlling or routing the information to the correct location while simultaneously directing every Nth frame to a display device for camera alignment. By "Nth frame" is meant whatever number is required for the user to obtain the desired alignment of the image being viewed. This desired alignment is obtained as above noted simply by moving the video camera until the user sees what he intends to record by video (or other camera) means.

In other embodiments, the means for communicating the image from the camera to the host computer changes as the embodiment changes. For example, if the capture card is located in the camera, the user would probably be communicating the information via the serial or parallel port. This is somewhat important because some of the digital cameras (single shot type cameras) have direct interfaces to the serial port for communicating captured images to computers. In this example, camera is not a video camera per se but it does capture still images. While the camera used in this disclosure will be for clarity a "video camera", this will include still cameras, a night vision camera, thermography camera, infra-red camera, etc. or any other suitable camera.

Embodiments of the invention will now be described, by way of example, with reference to the accompanying drawings, in which:
Figure 1 is a perspective view of the connection means of a video camera to the processor of the Mobile Assistant ®.
Figure 2 is a side view of a head-mounted display useful in the present invention.
Figure 3 is a perspective view of one use of the system of this invention or one embodiment of the invention illustrating the user video recording over the heads of a crowd.
Figure 4 is a side view of an embodiment of this invention where the video camera is connected to a Mobile Assistant ® and to head mounted display means.
Figure 5 is a perspective view of the processor of a Mobile Assistant ® having electrical or optical connection means to both the video camera and head mounted display in the system of this invention.

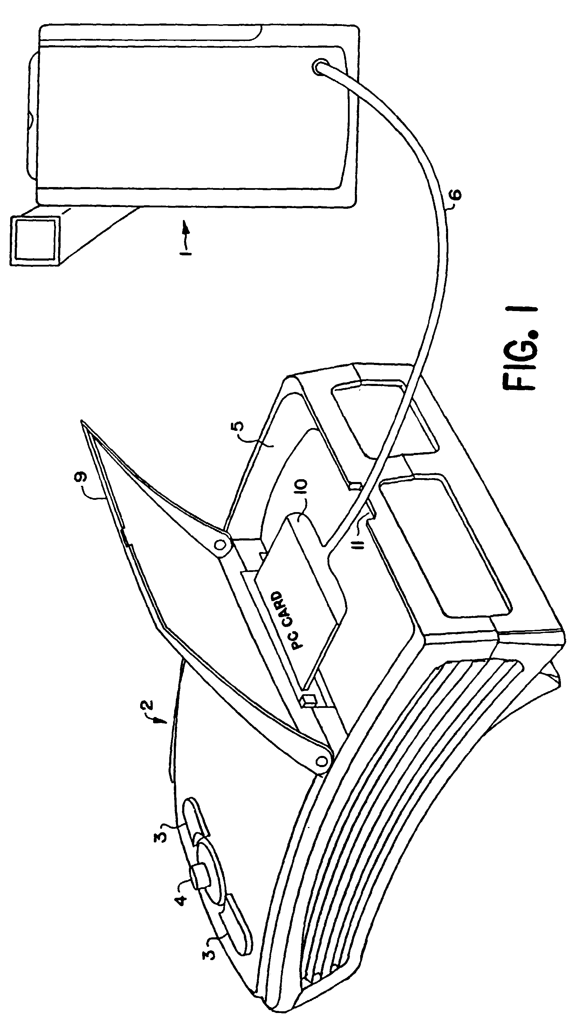

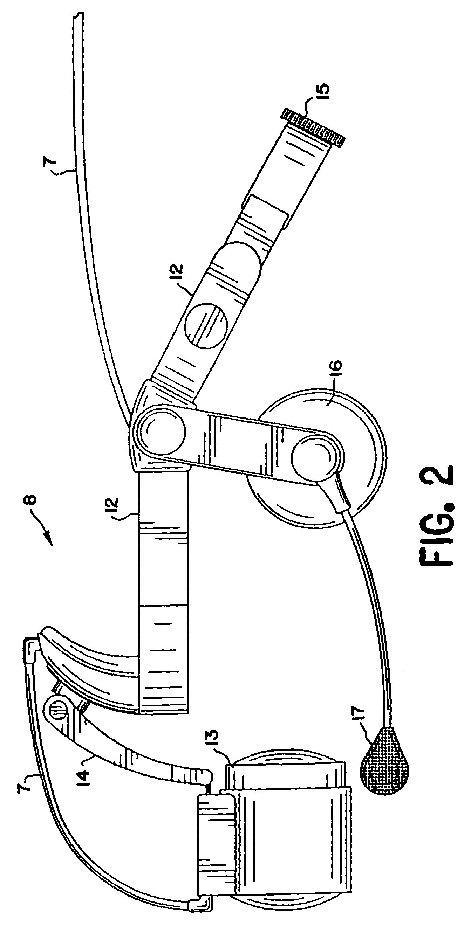

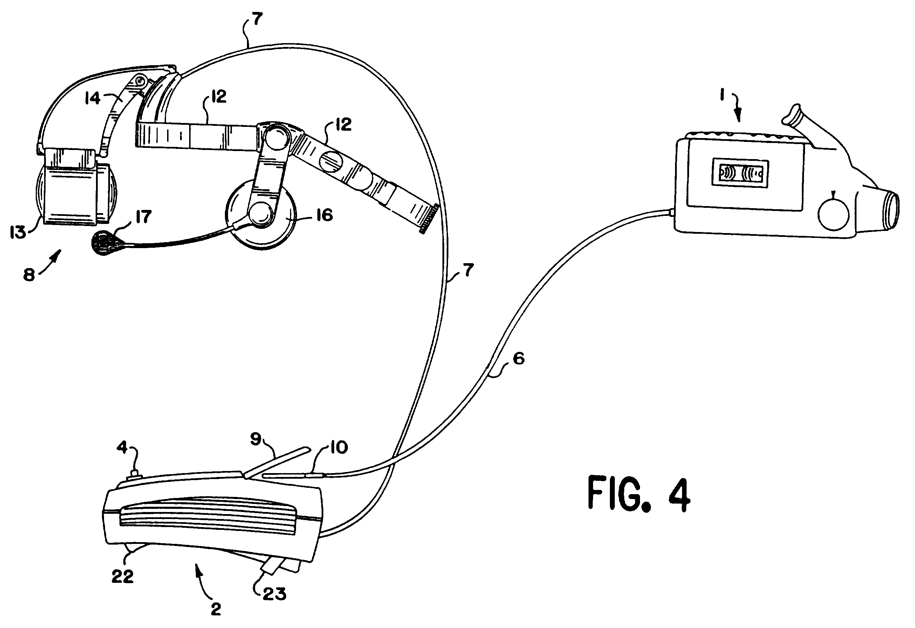

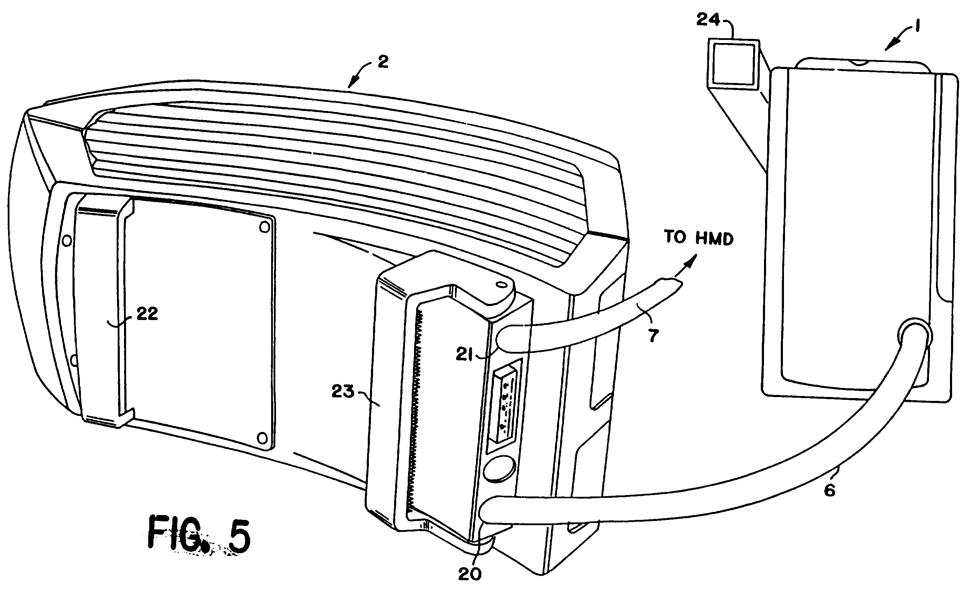

In figure 1 a processor 2 of a mobile computer is illustrated having control buttons 3 and a mouse lever 4 to be used when manual operation of the computer system is desired. However, in the present invention, the use of audio activation, eye tracking, electroencephalography or other hands-free activation is preferred; see EP-A-0,767,417 for suitable activation means. The processor 2 has a PCMCIA card housing 5 that holds a suitable PCMCIA (PC) card 10 useful in the present invention. Cable 6 extends from the processor 2 to a video (or other) camera 1, while cable 7 extends from an electrical outlet of the processor 2 to a head-mounted display (HMD) 8 or other monitor, see figures 4 and 5. A recessed PC card housing 5 keeps the card from being damaged once door 9 is closed. Slot 11 permits cable 6 to fit therethrough when door 9 is closed. Any suitable display may be used such as HMD 8 shown in figure 2.

In figure 2, a head-mounted display 8 comprises a narrow headband 12 with a pivotal eyepiece or cell 13 attached thereto. The eyepiece 13 is attached to a pivotal arm 14. The headband 12 is adjustable using adjusting means 15. Computer connection means or processor connection means 7 is shown attached to eyepiece 13 and extending in the rear of HMD 8 to processor 2. An earpiece or earphone 16 is also connected to headband 12 and microphone 17 is movably connected to earphone 16. As earlier noted, while audio activation is preferred, other suitable activation means may be used in the present invention including other hands-free activation means. EP-A-0,767,417 discloses some suitable activation means and other embodiments useful in the present invention. The video image transmitted to the HMD 8 is viewed by the user in eyepiece 13 and video camera 1 moved and redirected to record the desired image as viewed in eyepiece 13.

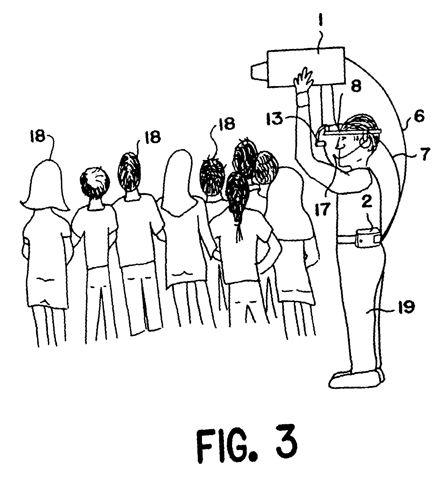

In figure 3 a user is shown holding a video camera 1 above the heads 18 of a crowd of people. Using his hands-free computer system comprising a display screen or eyepiece 13, a processor 2 and connecting cables 6 and 7, a user 19 can easily redirect the direction of camera 7 until the desired image is viewed on display screen 13. Obviously, from his position, the user 19 cannot view the desired image through a viewfinder in camera 1 but he can view the desired image through eyepiece 13 or move camera 1 until he does view the desired image through display screen 13. Processor 2 can have a PCMCIA card 10 that is programmed to record the Nth frame or view of the image desired. As noted, by Nth frame is meant whatever number of frames is necessary for the user to properly align the video camera 1.

In figures 4 and 5, the processor 2 of the Mobile Assistant ® may be connected to the video camera 1 via the PCMCIA card or via outlet connection 20 in processor 2. The HMD is connected to the processor 2 via cable 7 which also attaches to the processor 2 at a connection means 21. The processor 2 can include belt loops 22 and 23 which are used to attach the processor 2 to the waist (or other portion) of the user. Belt loop 23 can also be used as a movable stand when processor 2 is converted from a body worn unit to a stand alone computer as disclosed in EP-A-0,825,515. A body worn hands-free computer of this kind provides the computer means required in the system of this invention since mobility of the camera person would be impeded with a stationary computer or a laptop. As earlier noted, a Mobile Assistant ® adds the critical capabilities to this system not disclosed in the prior art, i.e. to communicate with other remote computers or systems such as Internet to display the image being recorded to other locations. These images can be analyzed as being recorded by the cameraman. If the mobile computer is the preferred voice activation computer, commands can be given by the cameraman or woman to other locations raising questions or requesting analysis of the images being displayed. Other benefits of having a computer managing remote cameras as opposed to just having a HMD communicating with a remote camera are the following:
(1) the computer can overlay other information while viewing the camera image;
(2) the computer can recall other captured images stored in the computer or remotely and compare to the image that is being viewed;
(3) the computer can communicate to other people using two-way voice while this option would require a separate unit;
(4) the computer can process the image to better define what is being viewed; and
(5) the computer has the ability to refer back to images that have been captured.

One of the benefits of having a computer with a HMD and a remote camera is that the user can recall information that is stored in a local or remote computer. This can be very useful when a user needs to assess whether an object that is currently being looked at has changed since the last time it was inspected. The ability to recall previously captured images and display the stored image with associated data about that image, concurrently with the image that is being looked at with the remote camera is not only desirable but necessary for determining if an object has changed over time. An example would be a maintenance inspector who needs to periodically evaluate the internal integrity of a pipe located inside a boiler room. The pipe inspection procedure requires that the pipe be inspected with a camera that has been snaked down an opening in the pipe. If a crack or a problem is encountered, the inspector can then call up information including images from the previous inspection of that pipe from a wearable computer or from a remote computer system (using the communications capability of the wearable computer). If the internal structure of the pipe has deteriorated from the last inspection then the appropriate maintenance should be performed. Similar uses other than inspection can also be easily accomplished using a mobile user-supported computer in the present system.

Thus, when eyepiece 24 in the video camera 1 cannot be used for any reason and remote means are needed to view an image, the present system provides convenient and mobile means to accomplish this. When it is needed to use the Mobile Assistant ® as part of a system for a remote viewing, the Mobile Assistant ® can be converted to use as disclosed in EP-A-0,767,417 and EP-A-0,825,515.

## Claims

1. A system for capturing and displaying a camera image to an apparatus apart from said camera, said system comprising image alignment means including a mobile computer in imagewise connection to a video camera, said mobile computer comprising hands-free activation means, said mobile computer comprising connection means for connection to said camera, a processor, and a user-supported display screen, said connection means comprising an electrical cable or fiber optics connected at one end to said processor, and at an opposite end to a connection receiving means in said video camera, said processor having means to transmit a camera image received via said connection means to said display screen, said processor having means to record every Nth frame of said image received and wherein said activating means are selected from the group consisting of audio activation, eye-tracking activation means, electroencephalography activation means and mixtures thereof.

2. A system for image alignment which comprises means for displaying a camera image to an apparatus separated from said camera, said system having image alignment means comprising a hands-free mobile wearable computer with hands-free activation means, said activation means selected from the group consisting of audio activation, eye-tracking activation means, brain activation means and mixtures thereof, said mobile wearable computer comprising means for connection to said camera, a processor, and a user-supported display screen, said means for connection to said camera comprising electrical connections or fiber optics connections at one end of said processor and at an opposite end to a connection receiving means in said video camera, said processor having means to transmit a camera image received via said connection to said display screen, said processor having means to record a set number of image frames of those received.

3. The system of claim 1 or claim 2 wherein said connection means are connected to a PCMCIA card in said processor.

4. The system of any one of claims 1 to 3 wherein said image alignment means comprises said activating means.

5. The system of any one of claims 1 to 4 wherein said mobile computer is a hands-free user-supported computer.

6. The system of any one of claims 1 to 5 wherein said display screen is on a head-mounted display supported by a user.

7. The system of any one of claims 1 to 6 wherein said Nth frame recorded is whatever number of frames is necessary to permit proper alignment of the video camera by a user.

8. The system of any one of claims 1 to 7 wherein said mobile computer has means for communication with a second location while said camera image is being captured.

9. The system of any one of claims 1 to 8 wherein said mobile computer has means for transmitting said camera image to a computer network.
